# FASCICULE DE BREVET EUROPEEN

(11) **EP 4 112 129 B1**
(45) Date de publication et mention de la délivrance du brevet: **09.08.2023**
(21) Numéro de dépôt: 22182116.8
(22) Date de dépôt: 30.06.2022
(51) Int. Cl.: A62C 3/08, B64C 7/02, B64D 25/00, F02C 7/25, A62C 35/02, B64D 45/00

(54) **ENSEMBLE COMPORTANT UNE STRUCTURE ET UN RÉSERVOIR FIXÉ À LA STRUCTURE ET CONTENANT UN FLUIDE EXTINCTEUR**
ANORDNUNG AUS EINER STRUKTUR UND EINEM BEHÄLTER, DER AN DER STRUKTUR BEFESTIGT IST UND EINE LÖSCHFLÜSSIGKEIT ENTHÄLT
ASSEMBLY COMPRISING A STRUCTURE AND A TANK ATTACHED TO THE STRUCTURE AND CONTAINING A FIRE EXTINGUISHING FLUID

(30) Priorité: 02.07.2021 FR 2107205
(43) Date de publication de la demande: 04.01.2023
(73) Titulaire: AIRBUS OPERATIONS (S.A.S.), 31060 Toulouse (FR)
(72) Inventeur: SOULIE, Adeline, 31060 TOULOUSE (FR); GELIOT, Jean, 31060 TOULOUSE (FR); CRUAUD PRIEUR, Solène, 31060 TOULOUSE (FR); GOUPIL, Frédéric, 31060 TOULOUSE (FR)
(74) Mandataire: Jardel, Marc Henry Philippe

(56) Documents cités:
- EP-A1- 3 305 372
- CN-U- 210 845 048
- CN-U- 211 584 982
- FR-A1- 3 105 177
- US-A1- 2010 230 122
- US-A1- 2019 151 690

## Description

La présente invention concerne un ensemble comportant une structure et un réservoir contenant un fluide extincteur et fixé à la structure, ainsi qu'un aéronef comportant au moins un tel ensemble.

Un aéronef comporte classiquement au moins une nacelle à l'intérieur de laquelle est disposé un moteur, par exemple du type turboréacteur. La nacelle et le moteur sont fixés à la structure de l'aéronef par l'intermédiaire d'un mât fixé sous l'aile de l'aéronef.

Afin d'éviter l'endommagement de la structure de l'aéronef lorsque le moteur prend feu, l'aéronef est équipé d'un système anti-incendie qui comporte classiquement deux réservoirs. Actuellement le fluide extincteur contenu dans les réservoirs est un produit appelé « halon ».

Pour des raisons de protection de l'environnement, de nouveaux produits vont progressivement remplacer le halon, mais pour obtenir le même résultat en termes de pouvoir d'extinction, il est nécessaire d'utiliser un volume plus important de ces nouveaux produits, ce qui entraîne également des réservoirs de plus grandes dimensions et une masse plus importante.

Les réservoirs sont classiquement montés dans un mât qui maintient la nacelle et l'intérieur d'un tel mât est un endroit relativement encombré. L'accroissement des réservoirs ne facilite pas leur montage à l'intérieur du mât et il est donc nécessaire de trouver un arrangement qui permet un montage facilité de chaque réservoir.

Un objet de la présente invention est de proposer un ensemble pour un aéronef, où ledit ensemble comporte une structure qui se fixe à une structure de l'aéronef et un réservoir contenant un fluide extincteur, et où l'installation du réservoir sur la structure est facilitée.

À cet effet, est proposé un ensemble selon la revendication 1. La demande de brevet FR3105177 décrit le préambule de la revendication 1. L'invention propose également un aéronef comportant un tel ensemble.

Un tel ensemble permet un retrait et une mise en place simples et rapides du réservoir.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
- la figure 1 est une vue de côté d'un aéronef selon l'invention,
- la figure 2 est une vue en perspective d'un ensemble selon l'invention avec le réservoir,
- la figure 3 est une vue en perspective d'une partie d'une structure de l'ensemble selon l'invention avec le réservoir démonté,
- la figure 4 est une vue en perspective et en coupe de la structure de la figure 2 par le plan IV,
- la figure 5 est une vue en coupe d'un système amortisseur mis en oeuvre dans l'ensemble selon l'invention,
- la figure 6 est une vue en perspective d'une partie de l'ensemble selon l'invention dans une position de prémontage, et
- la figure 7 est une vue en perspective d'une partie de l'ensemble selon l'invention dans une position de montage.

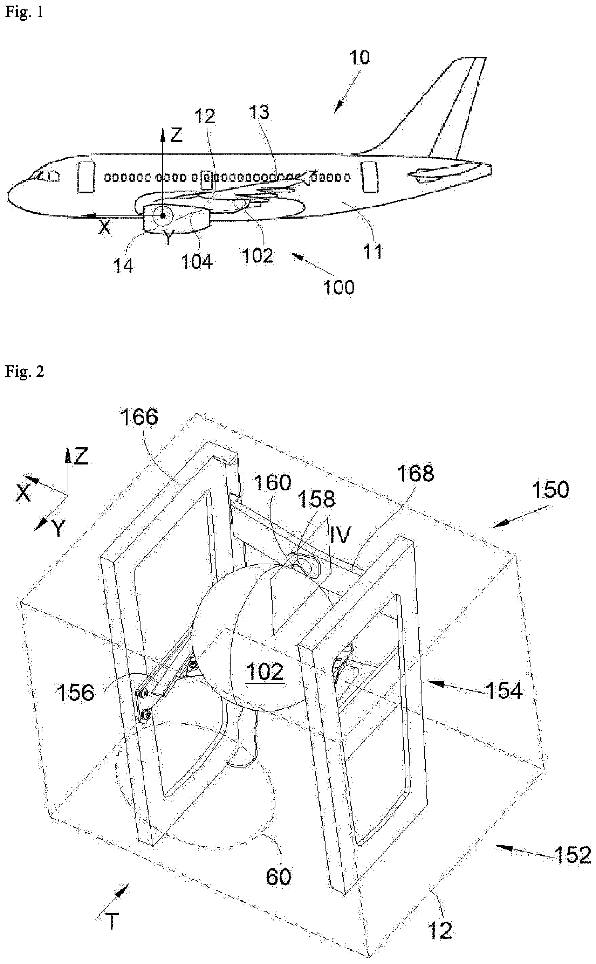

Dans la description qui suit, les termes relatifs à une position sont pris en référence à un aéronef en position normale de vol, c'est-à-dire comme il est représenté sur la figure 1.

Dans la description qui suit, et par convention, on appelle X la direction longitudinale du turboréacteur qui est parallèle à l'axe longitudinal de l'aéronef, on appelle Y la direction transversale qui est horizontale lorsque l'aéronef est au sol, et Z la direction verticale qui est verticale lorsque l'aéronef est au sol, ces trois directions X, Y et Z étant orthogonales entre elles.

La figure 1 montre un aéronef 10 qui comporte un fuselage 11 de chaque côté duquel est fixée une aile 13 qui porte un moteur 14 comme par exemple un turboréacteur double flux.

Pour chaque moteur 14, l'aéronef 10 comporte également un mât 12 qui assure la fixation du moteur 14 sous l'aile 13.

Pour chaque moteur 14, l'aéronef 10 comporte un système anti-incendie 100 qui comporte au moins un réservoir 102 et, pour chaque réservoir 102, une canalisation de décharge 104 qui s'étend entre ledit réservoir 102 et le moteur 14 soutenu par ledit mât 12.

La figure 2 montre un ensemble 150 selon l'invention qui comporte une structure 152 et un réservoir 102 fixé à la structure 152. L'ensemble 150 est logé à l'intérieur du mât 12 qui est matérialisé ici en traits mixtes. Classiquement, le réservoir 102 est rempli d'un fluide extincteur et est équipé d'une tête de décharge qui comporte un opercule qui ferme le réservoir 102 et une cartouche explosive, et où le goulot est fluidiquement connecté à la canalisation de décharge 104 à travers la tête de décharge. Lorsque cela est nécessaire, un ordre d'activation est envoyé à la cartouche explosive pour que celle-ci explose pour détruire l'opercule et ainsi libérer le fluide extincteur qui s'écoule dans la canalisation de décharge 104. Dans le mode de réalisation de l'invention présenté ici, il y a un seul ensemble 150, mais selon la quantité de fluide extincteur requis, il est possible de mettre en place plusieurs ensembles 150.

La structure 152 est fixée à l'intérieur du mât 12 par tous moyens de solidarisation appropriés comme par exemple des boulons. Le mât 12 présente une paroi percée d'une fenêtre 60 qui permet alternativement l'introduction ou le retrait du réservoir 102, en particulier lorsqu'il est nécessaire de le remplacer. L'introduction et le retrait du réservoir 102 s'effectuent selon une direction d'introduction T qui traverse la fenêtre 60 et qui est ici parallèle à la direction transversale Y.

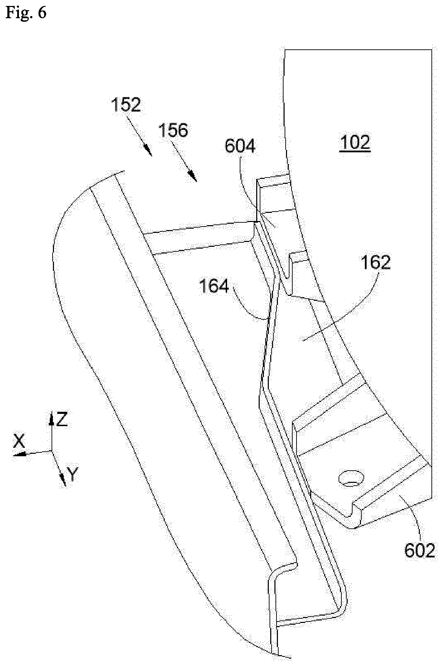

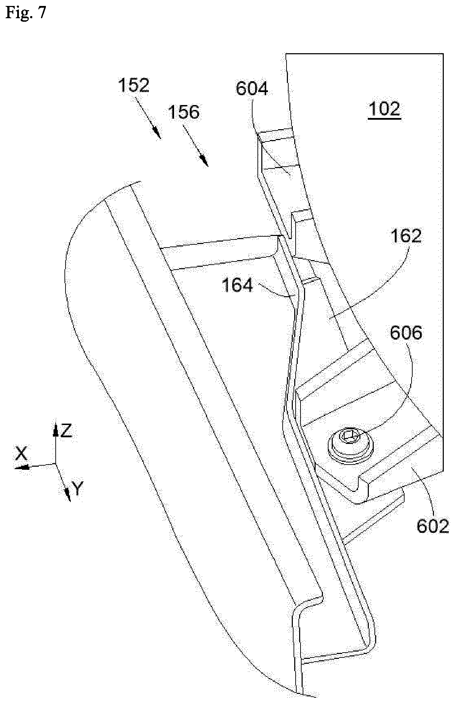

Les figures 6 et 7 montrent des détails de conception de l'invention et particulièrement du réservoir 102.

Le réservoir 102 prend ici la forme d'une sphère et il comporte deux pattes de fixation 602 et deux pattes d'appui 604 distinctes des pattes de fixation 602. Le réservoir 102 comporte également un plot 160 dont l'axe est parallèle à la direction d'introduction T.

Il y a une patte de fixation 602 et une patte d'appui 604 de chaque côté du réservoir 102, et ici symétriquement par rapport à un plan de symétrie vertical du réservoir 102.

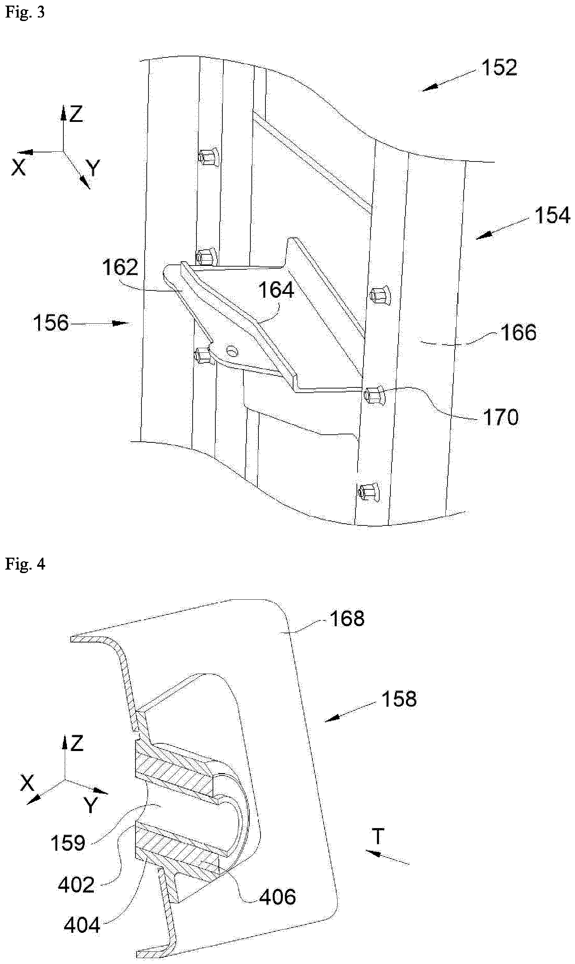

Les figures 3 et 4 montrent des détails de conception de l'invention et particulièrement de la structure 152.

La structure 152 comporte un châssis 154 fixé à l'intérieur du mât 12 à une structure dudit mât 12, deux supports 156 fixés au châssis 154 et un système d'emboîtement 158 également fixé au châssis 154 qui comporte un alésage 159 dont l'axe est parallèle à la direction d'introduction T et dont une ouverture est orientée vers la fenêtre 60.

Chaque support 156 comporte un plateau 162 fixé au châssis 154 et un guide 164 fixé au plateau 162. Les deux supports 156 sont disposés de part et d'autre du réservoir 102, ici de part et d'autre du plan de symétrie vertical du réservoir 102.

Lors de la mise en place du réservoir 102, celui-ci traverse la fenêtre 60 et il passe successivement d'une position de prémontage à une position de montage. Le réservoir 102 est ainsi mobile en translation parallèlement à la direction d'introduction T entre la position de prémontage et la position de montage lors de sa mise en place et inversement lors de son retrait.

La figure 6 montre la position de prémontage dans laquelle chaque patte de fixation 602 et chaque patte d'appui 604 reposent sur l'un des plateaux 162 et chaque guide 164 assure un guidage de la patte de fixation 602 et de la patte d'appui 604 associées audit plateau 162 transversalement par rapport à la direction d'introduction T, c'est-à-dire ici parallèlement à la direction longitudinale X. L'appui sur quatre pattes pendant le prémontage permet d'assurer une position stable du réservoir 102 et un tel guidage permet d'aligner le plot 160 et l'ouverture de l'alésage 159 du système d'emboîtement 158, même sans visibilité pour le technicien assurant la mise en place.

La figure 7 montre la position de montage dans laquelle chaque patte de fixation 602 repose sur le plateau 162 associé et le plot 160 est introduit dans l'ouverture de l'alésage 159 du système d'emboîtement 158. La fixation de chaque patte de fixation 602 au plateau 162 associé est réalisée par tous moyens de fixation appropriés comme par exemple des boulons 606. Dans cette position de montage, les pattes d'appui 604 ne reposent plus sur les plateaux 162. Le réservoir 102 est alors fixé à la structure 152 par les deux pattes de fixation 602 et le plot 160.

Un tel arrangement facilite la mise en place du réservoir 102 même lorsque celui-ci est de grandes dimensions.

Dans le mode de réalisation de l'invention présenté sur la figure 2, le châssis 154 comporte deux cadres 166 parallèles et disposés ici de part et d'autre du plan de symétrie du réservoir 102 et une traverse 168 qui relie les deux cadres 166 entre eux.

Chaque plateau 162 est fixé à l'un des cadres 166 et deux plateaux, et la traverse 168 porte le système d'emboîtement 158, c'est-à-dire que la traverse 168 est à l'opposé par rapport à la fenêtre 60.

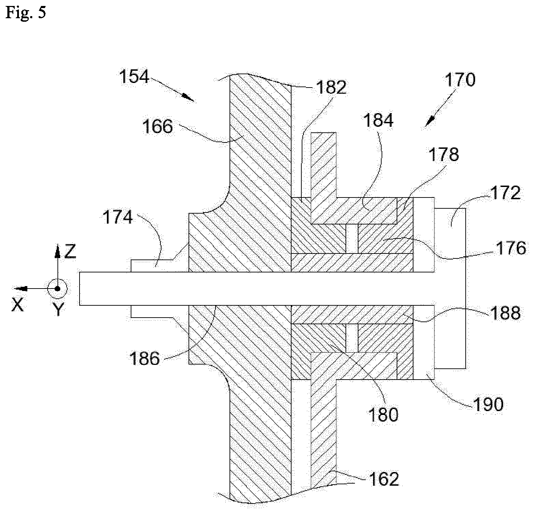

Pour limiter le transfert des vibrations environnantes au réservoir 102, chaque plateau 162 est fixé au châssis 154 par au moins un système amortisseur 170 qui sont ici au nombre de six par plateau 162 et dont un exemple est représenté de manière plus détaillée en coupe à la figure 5.

Le système amortisseur 170 comporte :
- une vis 172 avec une tête et une tige filetée dont l'axe est ici transversal par rapport à la direction d'introduction T,
- un écrou 174,
- une première bague 176 en élastomère qui présente un premier épaulement 178,
- une deuxième bague 180 en élastomère qui présente un deuxième épaulement 182,
- un palier extérieur 184 solidaire du plateau 162.

La première bague 176 est enfilée sur la tige filetée en présentant le premier épaulement 178 contre la tête de la vis 172, le palier extérieur 184 est enfilé sur la tige filetée et s'emmanche sur la première bague 176, la deuxième bague 180 est enfilée sur la tige filetée en s'emmanchant sous le palier extérieur 184 et en présentant le deuxième épaulement 182 à l'opposé du premier épaulement 178 pour être en appui contre le châssis 154, ici le cadre 166, la tige filetée est introduite dans un alésage 186 du châssis 154, ici du cadre 166, et l'écrou 174 est serré sur la tige filetée de l'autre côté du châssis 154 par rapport à la tête.

Après montage, le système d'amortisseur 170 comporte successivement la tête de la vis 172, le premier épaulement 178, le palier extérieur 184 emmanché sur la première bague 176 et la deuxième bague 180, le deuxième épaulement 182, le châssis 154 et l'écrou 174.

Le palier extérieur 184 est ainsi pris en sandwich entre le premier épaulement 178 et le deuxième épaulement 182.

Par la présence des deux bagues 176 et 180 qui entourent le palier extérieur 184, évitant le contact direct avec le châssis 154, les vibrations transmises au plateau 161 et donc au réservoir 102 sont très efficacement atténuées.

En outre, l'utilisation d'un guide et de fixations en matière élastomère permet, grâce à la relative élasticité conférée aux interfaces, d'absorber les tolérances de fabrication et d'assemblage et ainsi de garantir en toutes circonstances la possibilité de monter l'ensemble sur la structure d'un mât.

Dans le mode de réalisation de l'invention présenté ici, le système amortisseur 170 comporte en outre, un palier intérieur 188 qui est enfilé sur la tige filetée et sur lequel s'emmanche les deux bagues 176 et 180, et une rondelle 190 qui vient sous la tête pour agrandir la surface de contact avec le premier épaulement 178. Le contact entre le premier épaulement 178 et la tête s'effectue alors à travers la rondelle 190. Le palier inférieur 188 permet de limiter la compression des épaulements des bagues élastomères à une valeur prédéfinie, qui est réalisée lors du serrage au couple nominal du boulon. Les élastomères étant connus pour fluer sous charge permanente, cette bague garantit ainsi que le serrage est effectué sur un empilement de pièces rigides et donc l'absence de desserrage ultérieur de l'assemblage.

Pour limiter le transfert des vibrations, le système d'emboîtement 158 comporte également des moyens d'amortissement. Le système d'emboîtement 158 comporte :
- un tube intérieur 402 dont l'alésage forme l'alésage 159 pour loger le plot 160, c'est-à-dire que l'axe du tube intérieur 402 est parallèle à la direction d'introduction T,
- un tube extérieur 404 coaxial avec ledit tube intérieur 402, disposé à l'extérieur dudit tube intérieur 402, et fixé au châssis 154, ici à la traverse 168, et
- un tube intermédiaire 406 en élastomère qui est fixé entre le tube intérieur 402 et le tube extérieur 404.

## Revendications

1. Ensemble (150) comportant :
- une structure (152) comportant un châssis (154), deux supports (156) et un système d'emboîtement (158) fixé au châssis (154) et présentant un alésage (159) avec une ouverture et dont l'axe est parallèle à une direction d'introduction (T), où chaque support (156) comporte un plateau (162) fixé au châssis (154),
- un réservoir (102) contenant un fluide extincteur, et comportant deux pattes de fixation (602), deux pattes d'appui (604) distinctes des pattes de fixation (602), et un plot (160) dont l'axe est parallèle à la direction d'introduction (T), et
- des moyens de fixation (606),
**caractérisé en ce que** chaque support (156) comprend un guide (164) solidaire du plateau (162), et **en ce que** le réservoir (102) est arrangé pour être mobile en translation parallèlement à la direction d'introduction (T) entre une position de prémontage dans laquelle chaque patte de fixation (602) et chaque patte d'appui (604) reposent sur l'un des plateaux (162) et chaque guide (164) assure un guidage de la patte de fixation (602) et de la patte d'appui (604) associées audit plateau (162) transversalement par rapport à la direction d'introduction (T) pour aligner le plot (160) et l'ouverture de l'alésage (159) du système d'emboîtement (158), et une position de montage dans laquelle chaque patte de fixation (602) repose sur le plateau (162) associé, le plot (160) est introduit dans l'ouverture de l'alésage (159) du système d'emboîtement (158), les pattes d'appui (604) ne reposent plus sur les plateaux (162), et les moyens de fixation assurent la fixation de chaque patte de fixation (602) au plateau (162) associé.

2. Ensemble (150) selon la revendication 1, **caractérisé en ce que** chaque plateau (162) est fixé au châssis (154) par au moins un système amortisseur (170).

3. Ensemble (150) selon la revendication 2, **caractérisé en ce que** chaque système amortisseur (170) comporte :
- une vis (172) avec une tête et une tige filetée,
- un écrou (174),
- une première bague (176) en élastomère qui présente un premier épaulement (178),
- une deuxième bague (180) en élastomère qui présente un deuxième épaulement (182),
- un palier extérieur (184) solidaire du plateau (162),
où la première bague (176) est enfilée sur la tige filetée en présentant le premier épaulement (178) contre la tête, le palier extérieur (184) est enfilé sur la tige filetée et s'emmanche sur la première bague (176), la deuxième bague (180) est enfilée sur la tige filetée en s'emmanchant sous le palier extérieur (184) et en présentant le deuxième épaulement (182) à l'opposé du premier épaulement (178) et en appui contre le châssis (154), la tige filetée est introduite dans un alésage (186) du châssis (154) et l'écrou (174) est serré sur la tige filetée de l'autre côté du châssis (154) par rapport à la tête.

4. Ensemble (150) selon la revendication 3, **caractérisé en ce que** le système amortisseur (170) comporte un palier intérieur (188) enfilé sur la tige filetée et sur lequel s'emmanchent les deux bagues (176, 180).

5. Ensemble (150) selon l'une des revendications 3 ou 4, **caractérisé en ce que** le système amortisseur (170) comporte une rondelle (190) sous la tête de la vis (172).

6. Ensemble (150) selon l'une des revendications précédentes, **caractérisé en ce que** le système d'emboîtement (158) comporte :
- un tube intérieur (402) dont l'alésage forme l'alésage (159) pour loger le plot (160),
- un tube extérieur (404) coaxial avec ledit tube intérieur (402), disposé à l'extérieur dudit tube intérieur (402), et fixé au châssis (154), et
- un tube intermédiaire (406) en élastomère qui est fixé entre le tube intérieur (402) et le tube extérieur (404).

7. Aéronef (10) comportant un mât (12) dont une paroi présente une fenêtre (60), un ensemble (150) selon l'une des revendications précédentes, où le châssis (154) est fixé à l'intérieur du mât (12) et où l'ouverture de l'alésage (159) est orientée vers la fenêtre (60).

## Patentansprüche

1. Anordnung (150), umfassend:
- eine Struktur (152), die ein Gestell (154), zwei Stützen (156) und ein Stecksystem (158) umfasst, das am Gestell (154) befestigt ist und eine Bohrung (159) mit einer Öffnung und einer Achse, die parallel zu einer Einführungsrichtung (T) ist, aufweist, wobei jede Stütze (156) eine Platte (162) umfasst, die am Gestell (154) befestigt ist,
- einen Behälter (102), der eine Löschflüssigkeit enthält und zwei Befestigungslaschen (602), zwei von den Befestigungslaschen (602) verschiedene Stützlaschen (604) und einen Zapfen (160), dessen Achse parallel zur Einführungsrichtung (T) ist, umfasst, und
- Befestigungsmittel (606),
**dadurch gekennzeichnet, dass** jede Stütze (156) eine Führung (164) umfasst, die mit der Platte (162) fest verbunden ist, und dadurch, dass der Behälter (102) dazu eingerichtet ist, parallel zur Einführungsrichtung (T) translatorisch bewegbar zu sein zwischen einer Vormontageposition, in welcher jede Befestigungslasche (602) und jede Stützlasche (604) auf einer der Platten (162) ruht und jede Führung (164) ein Führen der Befestigungslasche (602) und der Stützlasche (604), die der Platte (162) zugeordnet sind, quer bezüglich der Einführungsrichtung (T) sicherstellt, um den Zapfen (160) und die Öffnung der Bohrung (159) des Stecksystems (158) zueinander auszurichten, und einer Montageposition, in welcher jede Befestigungslasche (602) auf der zugeordneten Platte (162) ruht, der Zapfen (160) in die Öffnung der Bohrung (159) des Stecksystems (158) eingeführt ist, die Stützlaschen (604) nicht mehr auf den Platten (162) ruhen und die Befestigungsmittel die Befestigung jeder Befestigungslasche (602) an der zugeordneten Platte (162) sicherstellen.

2. Anordnung (150) nach Anspruch 1, **dadurch gekennzeichnet, dass** jede Platte (162) durch mindestens ein Dämpfungssystem (170) am Gestell (154) befestigt ist.

3. Anordnung (150) nach Anspruch 2, **dadurch gekennzeichnet, dass** jedes Dämpfungssystem (170) umfasst:
- eine Schraube (172) mit einem Kopf und einem Gewindeschaft,
- eine Mutter (174),
- eine erste Hülse (176) aus Elastomer, die einen ersten Bund (178) aufweist,
- eine zweite Hülse (180) aus Elastomer, die einen zweiten Bund (182) aufweist,
- ein äußeres Lager (184), das mit der Platte (162) fest verbunden ist,
wobei die erste Hülse (176) auf den Gewindeschaft aufgeschoben ist, wobei sie den ersten Bund (178) am Kopf aufweist, das äußere Lager (184) auf den Gewindeschaft aufgeschoben ist und auf die erste Hülse (176) aufgesteckt ist, die zweite Hülse (180) auf den Gewindeschaft aufgeschoben ist, wobei sie unter das äußere Lager (184) gesteckt ist und den zweiten Bund (182) dem ersten Bund (178) gegenüberliegend und am Gestell (154) anliegend aufweist, der Gewindeschaft in eine Bohrung (186) des Gestells (154) eingeführt ist und die Mutter (174) auf dem Gewindeschaft auf der anderen Seite des Gestells (154) als der Kopf festgezogen ist.

4. Anordnung (150) nach Anspruch 3, **dadurch gekennzeichnet, dass** das Dämpfungssystem (170) ein inneres Lager (188) umfasst, das auf den Gewindeschaft aufgeschoben ist und auf das die zwei Hülsen (176, 180) aufgesteckt sind.

5. Anordnung (150) nach einem der Ansprüche 3 oder 4, **dadurch gekennzeichnet, dass** das Dämpfungssystem (170) eine Unterlegscheibe (190) unter dem Kopf der Schraube (172) umfasst.

6. Anordnung (150) nach einem der vorhergehenden Ansprüche, **dadurch gekennzeichnet, dass** das Stecksystem (158) umfasst:
- ein inneres Rohr (402), dessen Bohrung die Bohrung (159) zum Aufnehmen des Zapfens (160) bildet,
- ein mit dem inneren Rohr (402) koaxiales äußeres Rohr (404), das außerhalb des inneren Rohre (402) angeordnet und am Gestell (154) befestigt ist, und
- ein Zwischenrohr (406) aus Elastomer, das zwischen dem inneren Rohr (402) und dem äußeren Rohr (404) befestigt ist.

7. Luftfahrzeug, welches einen Pylon (12), von dem eine Wand ein Fenster (60) aufweist, und eine Anordnung nach einem der vorhergehenden Ansprüche umfasst, wobei das Gestell (154) im Inneren des Pylons (12) befestigt ist und wobei die Öffnung der Bohrung (159) dem Fenster (60) zugewandt ist.

## Claims

1. Assembly (150) having:
- a structure (152) having a chassis (154), two supports (156) and a nesting system (158) fastened to the chassis (154) and having a bore (159) with an opening and of which the axis is parallel to a direction of introduction (T), wherein each support (156) has a plate (162) fastened to the chassis (154),
- a reservoir (102) containing an extinguishing fluid, and having two fastening tabs (602), two bearing tabs (604) distinct from the fastening tabs (602), and a peg (160) of which the axis is parallel to the direction of introduction (T), and
- fastening means (606),
**characterized in that** each support (156) comprises a guide (164) as one with the plate (162), and **in that** the reservoir (102) is arranged so as to be able to move in translation parallel to the direction of introduction (T) between a premounting position in which each fastening tab (602) and each bearing tab (604) rest on one of the plates (162) and each guide (164) guides the fastening tab (602) and the bearing tab (604) that are associated with said plate (162) transversely with respect to the direction of introduction (T) so as to align the peg (160) and the opening of the bore (159) of the nesting system (158), and a mounting position in which each fastening tab (602) rests on the associated plate (162), the peg (160) is introduced into the opening of the bore (159) of the nesting system (158), the bearing tabs (604) no longer rest on the plates (162), and the fastening means fasten each fastening tab (602) to the associated plate (162).

2. Assembly (150) according to Claim 1, **characterized in that** each plate (162) is fastened to the chassis (154) by at least one damping system (170).

3. Assembly (150) according to Claim 2, **characterized in that** each damping system (170) has:
- a screw (172) with a head and a threaded shank,
- a nut (174),
- a first ring (176) made of elastomer that has a first shoulder (178),
- a second ring (180) made of elastomer that has a second shoulder (182),
- an outer bearing (184) as one with the plate (162), wherein the first ring (176) is threaded onto the threaded shank with the first shoulder (178) against the head, the outer bearing (184) is threaded onto the threaded shank and is fitted onto the first ring (176), the second ring (180) is threaded onto the threaded shank, being fitted beneath the outer bearing (184) and with the second shoulder (182) away from the first shoulder (178) and bearing against the chassis (154), the threaded shank is introduced into a bore (186) of the chassis (154) and the nut (174) is tightened on the threaded shank on the other side of the chassis (154) with respect to the head.

4. Assembly (150) according to Claim 3, **characterized in that** the damping system (170) has an inner bearing (188) threaded onto the threaded shank and on which the two rings (176, 180) are fitted.

5. Assembly (150) according to either of Claims 3 and 4, **characterized in that** the damping system (170) has a washer (190) beneath the head of the screw (172).

6. Assembly (150) according to one of the preceding claims, **characterized in that** the nesting system (158) has:
- an inner tube (402) of which the bore forms the bore (159) for housing the peg (160),
- an outer tube (404) coaxial with said inner tube (402), disposed outside said inner tube (402), and fastened to the chassis (154), and
- an intermediate tube (406) made of elastomer that is fastened between the inner tube (402) and the outer tube (404).

7. Aircraft (10) having a pylon (12) of which one wall has a window (60), an assembly (150) according to one of the preceding claims, wherein the chassis (154) is fastened inside the pylon (12) and wherein the opening of the bore (159) is oriented towards the window (60).
